# EUROPEAN PATENT APPLICATION

(11) **EP 1 803 659 A1**
(43) Date of publication of application: **04.07.2007**
(21) Application number: 06447127.9
(22) Date of filing: 08.12.2006
(51) Int. Cl.: B65D 81/26, G01N 33/52, G01N 33/543, B65D 75/34

(54) **Test strip package, container and manufacturing method for test strip package**

(30) Priority: 28.12.2005 JP 2005379632
(71) Applicant: SYSMEX CORPORATION, Kobe-shi, Hyogo 651-0073 (JP)
(72) Inventor: Imoarai, Takeshi, c/o Sysmex Corporation, Chuo-ku Kobe-shi Hyogo 651-0073 (JP); Oka, Akihide, c/o Sysmex Corporation, Chuo-ku Kobe-shi Hyogo 651-0073 (JP); Yamaguchi, Haruki, c/o Sysmex Corporation, Chuo-ku Kobe-shi Hyogo 651-0073 (JP)
(74) Representative: De Clercq, Ann G. Y.

(57) **Abstract**

The present invention is to present a test strip package that has less potential for accommodating a test strip (1) and a desiccant agent (3) at a location different from their proper location originally determined. The test strip package comprising: a test strip (1) for detecting an analyte contained in a sample; a desiccant agent (3); and a container comprising a first room (9) for housing the test strip (1), a second room (11) for housing the desiccant agent (3) and a cover (7) for sealing the first and second rooms, the second room (11) being communicated with the first room (9).

## Description

### FIELD OF THE INVENTION

The present invention relates to a test strip package, a container and a manufacturing method for a test strip package.

### BACKGROUND

In order to perform detection of an analyte in a sample (for example, pathogenic virus, protein, pharmacological material or the like), and judgment of properties of the sample (for example, hydrogen ion concentration or the like), a test strip is used.

Normally, a test strip has detection zone where exist substances which cause an antigen-antibody reaction with an analyte or substances which change their color upon contact with the analyte, or the like. When the detection zone makes contact with a sample, color or the like of the detection zone is changed, thereby allowing for detection of the analyte and judgment of properties of the sample.

Since substances existing at the detection zone of the test strip may change their nature due to humidity or the like, the test strip is packed in a package in bag-shape together with desiccant agents in plate-shape.

When taking out the test strip accommodated in the package in bag-shape, a designated portion of the package is cleaved to take out the test strip accommodated inside the package. Since the package accommodates the test strip at the same place of desiccant agent, it is necessary to accommodate the desiccant agent in plate-shape so that the desiccant agent may not come in contact with the detection zone of the test strip or the like, in packaging step of the test strip.

However, in the packaging step, if a test strip and desiccant agent in plate-shape are packed so as to allow for accidental contact of the desiccant agent with detection zone or the like of the test strip, the detection zone will be damaged, thereby giving influences on detection of the analyte.

The present invention has been developed in view of the above aspects and is to present a test strip package that has less potential for accommodating a test strip and a desiccant agent at a location different from their proper location originally determined.

### SUMMARY

A first aspect of the present invention is a test strip package comprising: a test strip for detecting an analyte contained in a sample; a desiccant agent; and a container comprising a first room for housing the test strip, a second room for housing the desiccant agent and a cover for sealing the first and second rooms, the second room being communicated with the first room.

A second aspect of the present invention is a container for housing a test strip and a desiccant agent, comprising: a first room for housing the test strip; and a second room for housing the desiccant agent and is communicated with the first room.

A third aspect of the present invention is a manufacturing method for a test strip package comprising steps of: providing a container assembly comprising a plurality of containers, wherein each container has a first room for housing a test strip and a second room for housing a desiccant agent; putting the test strip in each of the first rooms of the container assembly and the desiccant agent in each of the second rooms of the container assembly; and sealing the first and the second rooms of the container assembly so as to form a test strip package assembly.

### BRIEF DESCRIPTION OF THE DRAWINGS

FIG. 1 (a) is a plan view showing construction of a test strip package according to a first embodiment of the present invention, FIG. 1 (b) is a front view showing construction of the test strip package according to the first embodiment of the present invention, and FIG. 1(c) is a left-side elevation showing construction of the test strip package according to the first embodiment of the present invention;
FIG. 2 is a plan view showing construction of the test strip package according to the first embodiment of the present invention, illustrating a state before a cover 7 is heat-sealed;
FIG. 3 is a front view showing construction of a test strip to be accommodated in a test strip package according to the first embodiment of the present invention;
FIG. 4 is a plan view showing manufacturing step of the test strip package according to the first embodiment of the present invention;
FIG. 5 is a front view used for explanation of usage of the test strip package according to the first embodiment of the present invention;
FIG. 6 is a front view showing construction of the test strip package according to a second embodiment of the present invention;
FIG. 7 is a front view showing construction of the test strip package according to a third embodiment of the present invention;
FIG. 8 is a front view used for explanation of usage of the test strip package according to the third embodiment of the present invention; and
FIG. 9 is a front view used for explanation of usage of the test strip package according to the third embodiment of the present invention.

### DETAILED DESCRIPTION OF THE EMBODIMENT

Referring to drawings, embodiments of the present invention will be explained hereinafter. Drawings are used for convenience of explanations, and the scope of the present invention is not limited to embodiments shown in the drawings.

### 1. First embodiment

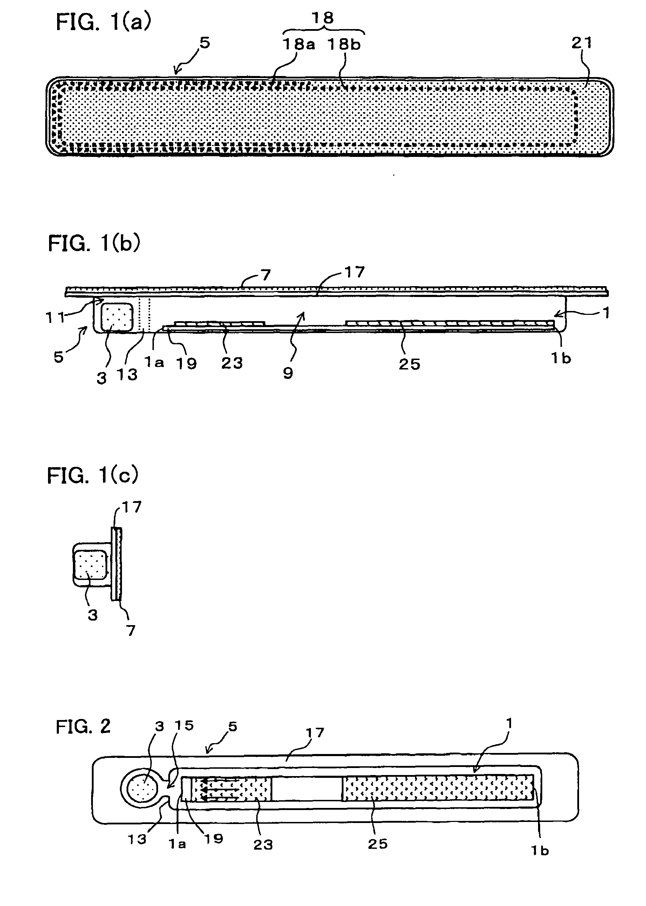

FIG. 1 (a) is a plan view showing construction of a test strip package according to a first embodiment of the present invention, FIG. 1 (b) is a front view showing construction of the test strip package according to the first embodiment of the present invention, and FIG. 1(c) is a left-side elevation showing construction of the test strip package according to the first embodiment of the present invention. FIG. 2 is a plan view corresponding to FIG. 1(a) and shows a state before a cover 7 is heat-sealed.

In the first embodiment, a test strip package for accommodating a test strip for immunochromatography used for detection of an analyte in a sample utilizing an antigen-antibody reaction will be explained. Chromatography is a method for separating and analyzing a substance utilizing a difference of its mobility in a carrier. Particularly, a method for detecting a substance utilizing antigen-antibody reaction is referred to as immunochromatography.

The test strip package according to the present embodiment comprises a test strip 1 for detecting an analyte in a sample, a desiccant agent 3, a container 5 for accommodating the test strip 1 and the desiccant agent 3, and a cover 7. The container 5 has a first room 9 for accommodating the test strip 1 and a second room 11 for accommodating the desiccant agent 3. The cover 7 seals off the first room 9 and the second room 11. The first room 9 and the second room 11 are communicated with each other via a communication portion 15, and it is possible for the desiccant agent 3 accommodated in the second room 11 to absorb moistures in the first room 9.

In this way, according to the present embodiment, the test strip 1 and the desiccant agent 3 are accommodated in the first room 9 and the second room 11 of the container 5, respectively. Therefore, a place for accommodating the test strip 1 and a place for accommodating the desiccant agent 3 are definitely distinguished, and a possibility=that the test strip 1 and the desiccant agent 3 are accommodated at a location other than their proper location originally determined is lowered, and the test strip 1 and the desiccant agent 3 are capable of being accommodated properly.

The container 5 further comprises a partition portion 13 which limits a movement of the desiccant agent 3 to the first room 9 and a movement of the test strip 1 to the second room 11.

The container 5 has a peripheral portion 17 and the cover 7 is heat-sealed to the peripheral portion 17. With this configuration, the cover 7 can be heat-sealed easily to the container 5. Dotted line in FIG. 1 (a) shows an attached portion 18.

The test strip 1 has a sample contacting portion 19 at one end 1a thereof and is accommodated in the first room 9 so that the sample contacting portion 19 may be positioned on the second room 11 side of the first room 9. A peeling start portion 21 of the cover 7 is arranged at a position corresponding to other end 1b side (i.e., first room 9 side of the container 5) of the test strip 1. With this configuration, the cover 7 is being peeled from the peeling start portion 21 of the cover 7 and this prevents the sample contacting portion 19 of the test strip 1 from being touched by fingers. When cleaving conventional package to take out a test strip from the package, it is frequently caught by test strip accommodated inside, and unsealing is not performed satisfactorily, and the test strip is hardly taken out. However, with said configuration, a package can be unsealed with ease due to that the peeling start portion 21 is provided to the cover 7, thereby resulting in ease of taking out of the test strip 1.

The attached portion 18 comprises a first attached portion 18a and a second attached portion 18b. The first attached portion 18a is formed widely at one end 1a side of the test strip 1, as illustrated by two dotted lines in FIG. 1 (a). The second attached portion 18b is formed narrowly at other end 1b side of the test strip 1 as illustrated by one dotted line in FIG. 1 (a) . The cover 7 is heat-sealed at the first attached portion 18a more strongly than the second attached portion 18b. Therefore, the cover 7 is capable of being peeled substantially at the second attached portion 18b only. With this configuration, when taking out the test strip 1 from the container 5, it is possible to prevent such an occasion that the desiccant agent 3 comes out from the package. Further, touching the sample contacting portion 19 of the test strip 1 with fingers can be prevented positively.

To the test strip 1 are pasted a first and a second protection seals 23, 25. An arrow is drawn on the first protection seal 23 showing a direction of the sample contacting portion 19.

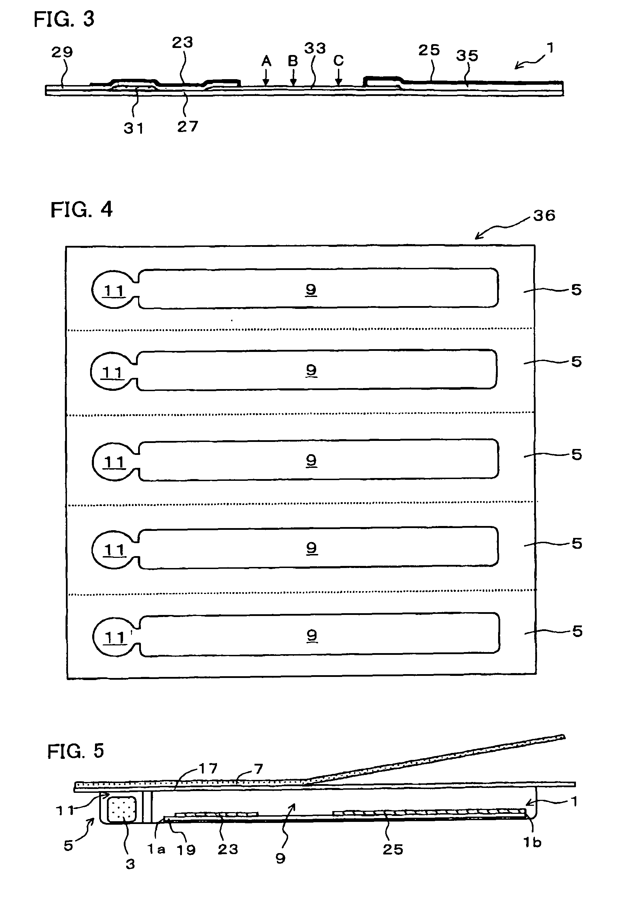

Now, detailed construction of the test strip 1 will be explained hereafter using FIG. 3. Fig. 3 is a front view showing construction of a test strip 1.

The test strip 1 comprises a sample contacting member 29 made of unwoven cloth (rayon) , a label holding member 31 made of unwoven cloth (glass fiber), a chromatography membrane 33 composed of madreporic body made of nitrocellulose, and an absorption member 35 composed of unwoven cloth (cellulose) , which are mounted on a substrate 27 composed of a plastic plate having an adhesion layer on the surface thereof. The sample contacting member 29 functions as a sample contacting portion which is immersed into a sample. The label holding member 31 is disposed in contact with the sample contacting member 29 and functions as a label holding portion for holding a labeling substance that causes an antigen-antibody reaction with an analyte in the sample. The sample contacting member 29 is disposed so as to cover the label holding member 31 and disposed in contact with the chromatography membrane 33. The label holding member 31 is disposed being isolated from the chromatography membrane 33. The chromatography membrane 33 has a detection zone to which is fixed an immobilizing material which causes an antigen-antibody reaction with an analyte. The absorption member 35 is disposed so as to contact with the chromatography membrane 33. The absorption member 35 absorbs samples which passed through the detection zone. The first and second protection seals 23, 25 cover respectively the sample contacting member 29 and the absorption member 35.

To the chromatography membrane 33 are formed, in the order from upstream, a first detection zone A, a second detection zone B and a control zone C in line-shape. A first labeling substance, a second labeling substance and control labeling substance are held to the label holding member 31. To the first detection zone A, second detection zone B and control zone C are immobilized, as the immobilizing material, antiinfluenza A virus antibody, antiinfluenza B virus antibody (hereinafter referred to respectively as "Anti-Flu A antibody", " Anti-Flu B antibody"), and biotin, respectively. The first labeling substance and second labeling substance are anti-Flu A antibody and anti-Flu B antibody being labeled by blue latex particles, respectively, and the control labeling substance is avidin that is labeled by red latex particles. The anti-Flu A antibody and anti-Flu B antibody bind, by antigen-antibody reaction, respectively to influenza A virus that is the first analyte and to influenza B virus that is the second analyte (hereafter referred to respectively as "Flu A virus", " Flu B virus").

When a sample contains Flu A virus, labeled anti-Flu A antibody, that is present in the label holding member 31, recognizes a specific site of Flu A virus and forms a complex by binding thereto by antigen-antibody reaction. Next, anti-Flu A antibody, that is present in the chromatography membrane 33, recognizes another site of Flu A virus and arrests a complex. When a complex is arrested, a blue line appears to the first detection zone A, and Flu A virus is detected visually.

Further, although avidin is not arrested by anti-Flu A antibody, anti-Flu B antibody which are present in the chromatography membrane 33, it specifically binds to biotin, and therefore, is arrested by biotin immobilized to the control zone C. When avidin is arrested, a red line appears to the control zone C, and it is visually confirmed that avidin reached the control zone C. Since the control zone C is provided downstream of the first detection zone A and second detection zone B, it is confirmed that, by confirming a red line, the sample passed through the first detection zone A and second detection zone B.

Next, the desiccant agent 3 will be explained. The desiccant agent 3 can be produced by kneading a resin material and a hygroscopic material, and by molding it to a block shape such as cylindrical column or rectangular column. For resin materials, polystyrene, PP (polypropylene) , PVC (vinyl chloride), PET (polyethylene terephthalate), PC (polycarbonate), PE (polyethylene) , nylon or the like (preferably thermoplastic) may be used. For hygroscopic materials, silica gel, calcium chloride, molecular sieve, silica dioxide, alumina, zeolite or the like may be used.

Next, referring to FIG. 4, a method for manufacturing a test strip package (hereafter referred also to as "package") will be explained. FIG. 4 is a plan view showing manufacturing step of the package according to the present embodiment.

First, as shown in FIG. 4, a container assembly 36 in which a plurality of containers 5 are connected consecutively is produced. The container assembly 36 can be produced by heating a material in sheet-form and molding (drawing molding) it. For materials in sheet-form, those which are heat-sealable are preferred. For materials in sheet-form, those which hardly allow moisture to pass through are preferred. For materials in sheet-form, those having a first resin layer, aluminum layer and a second resin layer being laminated may be used. The first resin layer and second resin layer may be formed by resin materials (preferably thermoplastic) such as polystyrene, PP (polypropylene), PVC (vinyl chloride), PET (polyethylene terephthalate), PC (polycarbonate), PE (polyethylene), nylon or the like. A material in sheet-form may be single layer of previously-mentioned resin materials or aluminum. Further, it is possible to produce the container assembly 36 by pouring molten resin material into a die for molding (e.g., injection molding). Further, the container assembly 36 may be molded by pressing a metal material (e.g., aluminum) in sheet-form using a die. Next, the test strip 1 is put in each of the first room 9 of the container assembly 36, and the desiccant agent 3 is put in each of the second room 11.

Further, each of the first room 9 and each of the second room 11 are sealed by the cover 7 which has a size substantially equivalent to that of the container assembly 36. By cutting the cover 7 and container assembly 36 at a position corresponding to dotted line portion in FIG. 4, a plurality of packages can be obtained. Alternatively, a package assembly, in which a plurality of packages before cutting are connected consecutively, may be provided as it is. In this case, the package assembly is preferably produced so that a user of the test strip 1 could separate one package from the package assembly at the position corresponding to dotted line portion of the container assembly 36 shown in FIG. 4. Specifically, such a package assembly can be produced using, for example, a container assembly 36 in which dotted line portion shown in FIG. 4 is molded to be thinner than other portion or a container assembly 36 having a perforated line at dotted line portion. Besides, the cover 7 covering the container assembly 36 is preferably composed to be separated easily at dotted line portion by providing a cut line or a perforated line at the position corresponding to dotted line portion shown in FIG. 4. With this consideration, one package can be separated from the package assembly more easily.

When packages are mass produced in efficient fashion, above-mentioned method of manufacturing is particularly preferable.

With conventional method, a step for accommodating a test strip and desiccant agent in a package, and a step for sealing the package are carried out in series for every package. According to the method of manufacturing of the present embodiment, a plurality of packages can be produced in efficient fashion, and therefore, production efficiency is improved and manufacturing costs are reduced.

Furthermore, by providing a certain degree of rigidity to the container 5 or container assembly 36, it is possible to prevent the test strip 1 accommodated inside from being curved due to external force.

The first room 9 and second room 11 of the container 5 have a size capable of accommodating the test strip 1 and desiccant agent 3, respectively. A difference in the length between the first room 9 and the test strip 1 is preferably not less than 1 cm (more preferably 2 cm) . In this case, by tilting the container 5 so that one end 1a of the test strip 1 may be located lower, a clearance is generated to other end 1b side of the test strip 1, thereby allowing easy taking-out of the test strip 1. Besides, said difference in the length is preferably less than 4 cm (more preferably 3 cm) to prevent the container 5 from becoming excessively long.

The partition portion 13 is provided between the first room 9 and second room 11 to limit a movement of the desiccant agent 3 into the first room 9 and a movement of the test strip 1 into the second room 11. With this configuration, contact of the test strip 1 with the desiccant agent 3 could be avoided. The partition portion 13 may be formed so that side wall of the container 5 is protruded towards inside of the container 5, or formed so that bottom wall of the container 5 is protruded towards inside of the container 5. The peripheral portion 17 may be formed to be integrated with the first room 9 and second room 11 (portion forming these members is referred to as "accommodation container body"), or separate member that is used as the peripheral portion 17 may be attached to the accommodation container body after the accommodation container body is formed.

Next, the cover 7 will be explained. For the cover 7, materials in sheet-form similar to those as used for the container 5 may be used. Composition and thickness of the material in sheet-form may be same as or different from those of the container 5. The cover 7 may be heat-sealed to the container 5, or adhered to the container 5 by using adhesive agent. The attached portion 18 is formed by heat-sealing or adhesion. The attached portion 18 may have the first attached portion 18a and second attached portion 18b each having different heat-sealing strength or whole heat-sealing strength may be same. Further, the first attached portion 18a may be disposed at a place where the cover 7 covers the sample contacting portion 19 of the test strip 1 (one end 1a of test strip 1 by another expression), or may be disposed at a place where the cover 7 covers the second room 11. In another embodiment, strength of the first attached portion 18a may be improved by, when forming the first attached portion 18a, increasing a temperature, a time or a pressure of the heat-sealing, or using an adhesive agent developing higher adhesive strength. In this case, width of the first attached portion 18a and second attached portion 18b may be designed to be identical. The first attached portion 18a may be formed by heat-sealing and the second attached portion 18b may be formed by adhesion, or vice versa.

Next, one example of usage of the test strip package according to the present embodiment will be explained.

First, the peeling start portion 21 of the cover 7 is grasped and the second attached portion 18b is peeled off. By these manipulations, as shown in FIG. 5, the cover 7 is peeled off at the second attached portion 18b, and it is now possible to take out the test strip 1 from the container 5 by grasping other end 1b side of the test strip 1. On this occasion, since the cover 7 is not being peeled off at one end 1a side of the test strip 1, there is no possibility that one touches accidentally the sample contacting portion 19 of the test strip 1. Further, since a portion covering the second room 11 of the cover 7 is not peeled off, the desiccant agent 3 would not come out when taking out the test strip 1 from the container 5. The test strip 1 taken out is inserted into a test container (not shown), into which is filled a sample, so that the sample contacting portion 19 may be immersed into the sample. It is then left for approximately 10 to 20 min and is used to check the presence or absence of an analyte.

Further, another embodiment of the present invention will be explained hereinafter.

Analytes are not limited specifically, and cells (for example, bacteria, protists, fungi or the like), viruses, proteins, polysaccharides, drugs (for example, narcotics, psychostimulant or the like), or the like are mentioned. For example, in addition to foregoing influenza virus, parainfluenza viruses, RS viruses, *Mycoplasma pneumoniae,* rotavirus, calicivirus, coronavirus, adenovirus, enterovirus, herpesvirus, human immunodeficiency virus, hepatitis virus, pathogenic virus of severe acute respiratory syndrome, coli *bacillus, Staphylococcus* aureus, *Streptococcus pneumoniae, Streptococcus pyogenes,* mararia parasite, others, pathogenic organisms for various disorders such as alimentary diseases, central nervous system diseases, hemorrhagic fever or the like, metabolic products thereof, carcinoembryonic antigen, tumor markers such as Cyfra or the like, hormone, oxypurine, glucose, bilirubin, uric acid, urobilinogen, leukocytes, benzodiazepines, cocaine system narcotics, morphine system narcotics, cannabis, tricyclic antidepressant drug, barbituric acids, phencyclidines or the like are exemplified.

The substrate 27 is to dispose thereon the foregoing members such as sample contacting member 29 and label holding member 31 or the like, and various materials such as papers or glasses may be used in addition to plastics. The sample contacting member 29 may be formed by various materials such as glass fiber or cellulose fiber in addition to rayon. The label holding member 31 may be formed by various materials such as cellulose fiber or the like in addition to glass fiber. The chromatography membrane 33 may be formed by various materials such as nylon (e.g. modified nylon in which is introduced amino group that may have carboxyl group or alkyl group as the substitution group), polyvinylidene-difluoride (PVDF), cellulose acetate or the like, in addition to cellulose nitrate. The absorption member 35 may be formed by various materials such as glass fiber or the like in addition to cellulose. For the sample contacting member 29, the label holding member 31, the chromatography membrane 33 and the absorption member 35, materials with various structures which are capable of developing a sample by capillary phenomenon may be used in addition to unwoven cloth or madreporic body. Further, a developing member composed of rayon, glass fiber or cellulose fiber may be provided between the label holding member 31 and the chromatography membrane 33. With this configuration, elution of labeling substance in the label holding member 31 is accelerated, thereby allowing prompt measurements.

The chromatography membrane 33 may include only one detection zone or more than two depending on types of the analyte. Further, the chromatography membrane 33 may not include control zone. The detection zone and the control zone may not be in line-shape and may be formed, for example, to circular form or rectangular form. The label holding member 31 may hold only one type of labeling substance or more than two types. Further, the label holding member 31 may not hold labeling substance for control purpose. The labeling substance may be labeled by latex particles other than blue and red, metal colloid such as gold, dye molecule or the like. In a case more than two labeling substances are used, each labeling substance may be labeled by different colors or by the same color. Further, the labeling substance and the labeling substance for control purpose may be labeled by colors different each other or by the same color.

Immobilization substances and labeling substances are not limited as long as they are able to bind specifically to above-mentioned analytes. For example, various antibodies and antigens may be used. In other words, when an analyte is an antigen, antibodies which cause an antigen-antibody reaction with this antigen may be used for immobilization substances and labeling substances; when an analyte is as antibody, antigens which cause an antigen-antibody reaction with this antibody, or antibodies which cause an antigen-antibody reaction with the antibody that is an analyte, may be used for immobilization substances and labeling substances. Besides, when detecting saccharide or glycoprotein, lectin may be used for immobilization substances and labeling substances.

An immobilization substance at the control zone may be avidin and a labeling substance for control purpose may be biotin. Further, an immobilization substance at the control zone and a labeling substance for control purpose may be other than the combination of biotin and avidin. For example, a combination that causes binding by antigen-antibody reaction may be used. For example, an antigen is used for labeling substance for control purpose and an antibody that causes an antigen-antibody reaction with this antigen is used for immobilization substance of control zone. The reverse of this combination may be used. For labeling substances for control, those which do not cause an antigen-antibody reaction with analyte and immobilization substance of detection zone are used.

Meanwhile, composition of the test strip 1 is not limited as long as it is capable of detecting an analyte in the sample.

The container according to the present embodiment may accommodate test strip for chromatography which does not use antigen-antibody reaction and test strip which does not use chromatography, but not test strip for immunochromatography as used in the first embodiment, together with desiccant agent.

For other concrete examples of the test strip, urine test paper, water quality inspection paper, formaldehyde measurement paper, humidity indicator card, pH-test paper, litmus paper or the like are mentioned.

For example, by using a urine test paper, urinary glucose, oxypurine, protein (e.g., urinary albumin) or the like can be detected quantitatively or qualitatively. When detecting urinary glucose, it is possible to cause the detection zone of chromatography membrane of test paper to hold enzymes (glucose oxidase, peroxidase or the like) and chromogen. When detecting urinary protein, it is possible to cause the detection zone to hold buffering agent and pH indicator (e.g., tetrabromo phenol blue or the like).

By using water quality inspection paper, it is possible to detect pH, hardness, chlorine, nitrite-nitrogen, nitrate-nitrogen, ammonium-nitrogen, phosphoric ion, metallic ion (e.g., copper ion, ferric ion or the like) or the like of a sample (e.g., well water, tap water, river water or the like).

By using formaldehyde measurement paper, it is possible to detect formaldehyde generated from building materials or adhesive agents.

By using humidity indicator card, it is possible to detect humidity of a sample (gaseous matter).

By using litmus paper, it is possible to make judgment if a sample is acid, neutral or alkali.

### 2. Second embodiment

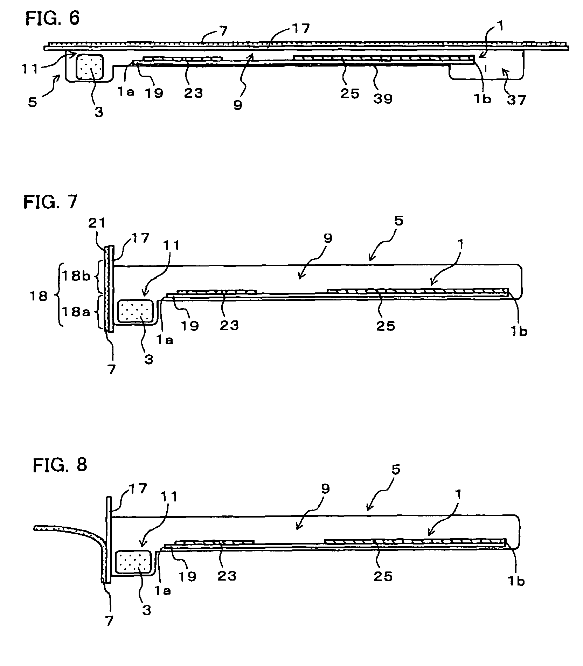

FIG. 6 is a front view showing construction of the test strip package according to a second embodiment of the present invention.

With the test strip package according to the present embodiment, depth of the second room 11 is deeper than depth of the first room 9. The first room 9 has an elongated shape and one end thereof is communicated with the second room 11. Further, a take-out portion 37 is provided at other end of the first room 9. A placing portion 39 for placing the test strip 1 is provided between one end and other end of the first room 9. A depth of the take-out portion 37 is deeper than the placing portion 39. The depth of the first room 9 denotes a depth at a portion where the test strip 1 is placed.

According to the present embodiment, a movement of the desiccant agent 3 to the first room 9 is prevented by a step provided between the first room 9 and the second room 11. Further, when the test strip 1 moves towards the second room 11, the test strip 1 collides with the desiccant agent 3 and is unable to proceed further, and consequently, a movement of the test strip 1 to the second room 11 is also prevented. A partition portion between the first room 9 and the second room 11 may be provided or may not be provided. Besides, since the first room 9 can be configured to be comparatively shallower, turning of the test strip 1 in the first room 9 can be prevented.

Further, by inserting a finger into the take-out portion 37 and raising the test strip 1 from underneath, it is possible to take out the test strip 1 easily from the container 5. To allow easy insertion of the finger under the substrate of the test strip 1, a length of the take-out portion 37 is preferably not less than 1.5 cm (more preferably 2 cm) . Besides, to prevent the container 5 from becoming too long, the length of the take-out portion 37 is preferably not more than 4 cm (more preferably 3 cm).

Details explained in the first embodiment are basically applicable to the present embodiment.

### 3. Third embodiment

FIG. 7 is a front view showing construction of the test strip package according to a third embodiment of the present invention.

With the test strip package according to the present embodiment, an opening of the container 5 and the peripheral portion 17 are provided at the position where one end 1a side of the test strip 1 (sample contacting portion 19 side) is elongated in longitudinal direction. Both the first room 9 and the second room 11 are faced with an opening of the container 5. The cover 7 is, in a similar fashion as the first embodiment, heat-sealed to the peripheral portion 17 by the attached portion 18 composed of the first attached portion 18a and the second attached portion 18b. The first attached portion 18a is provided at a position where the cover 7 covers the second room 11. The cover 7 has the peeling start portion 21 at the first room 9 side. The container 5 according to the present embodiment can be produced from resin materials shown in the first embodiment by die molding or the like. Details explained in the first embodiment are basically applicable to the present embodiment.

Next, one example of usage of the test strip package according to the present embodiment will be explained.

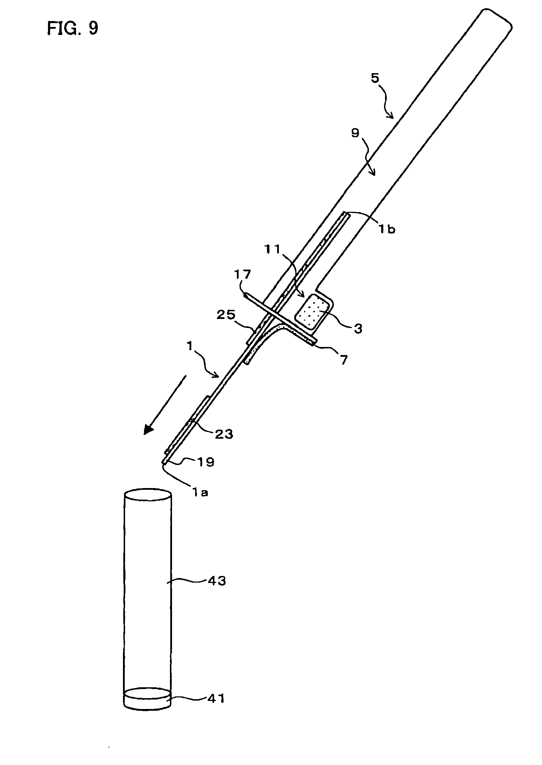

First, the peeling start portion 21 of the cover 7 is grasped and the second attached portion 18b is peeled off. By these manipulations, as shown in FIG. 8, the cover 7 is peeled off at the second attached portion 18b. Next, as shown in FIG. 9, a test container 43 to which is filled a sample 41 is prepared, and the container 5 is tilted towards the test container 43 so that one end 1a of the test strip 1 may be located lower. By these manipulations, the test strip 1 slips out from the container 5 and is inserted into the test container 43. The test strip 1 is left for approximately 10 to 20 min, and is used to check the presence or absence of an analyte in the sample 41. In this way, according to the present embodiment, it is possible to insert the test strip 1 into the test container 43 by simply tilting the container 5. Namely, since a test strip can be taken out from the container 5 without touching the test strip 1, contamination of the test strip 1 can be prevented.

Meanwhile, "taken out" as used in the specification also includes to move the test strip 1 from inside to outside of the container 5 by tilting the container 5 or the like, not only to move the test strip 1 from inside to outside of the container 5 by grasping the test strip 1 by fingers or the like from the container 5.

Various features shown in the foregoing embodiments may be combined each other. In a case where a plurality of features are involved in one embodiment, one or more than one features may be picked up appropriately therefrom and be employed, alone or in combination, in the test strip package of the present invention.

The foregoing detailed description and accompanying drawings have been provided by way of explanation and illustration, and are not intended to limit the scope of the appended claims. Many variations in the presently preferred embodiments illustrated herein will be obvious to one of ordinary skill in the art, and remain within the scope of the appended claims and their equivalents.

## Claims

1. A test strip package comprising:
a test strip for detecting an analyte contained in a sample;
a desiccant agent; and
a container comprising a first room for housing the test strip, a second room for housing the desiccant agent and a cover for sealing the first and second rooms, the second room being communicated with the first room.

2. The test strip package of Claim 1, wherein
the container further comprises a communication portion arranged between the first room and the second room.

3. The test strip package of Claim 1 or Claim 2, wherein
a depth of the second room is deeper than that of the first room.

4. The test strip package of any one of Claims 1 to 3, wherein
the first room comprises a placing portion for placing the test strip and a take-out portion arranged at one end side of the placing portion, and wherein other end of the placing portion is communicated with the second room, and a depth of the take-out portion is deeper than that of the placing portion.

5. The test strip package of any one of Claims 1 to 4, wherein
the container has a peripheral portion and the cover is heat-sealed to the peripheral portion.

6. The test strip package of any one of Claims 1 to 5, wherein
the test strip has a sample contacting portion at one end thereof and is accommodated in the first room so that the sample contacting portion is positioned on the second room side of the first room.

7. The test strip package of Claim 6, wherein
the cover has a peeling start portion for peeling the cover at a position corresponding to other end side of the test strip.

8. The test strip package of Claim 5, wherein
one end portion on the second room side of the cover is heat-sealed more strongly than other end portion on the first room side of the cover.

9. The test strip package of any one of Claims 1 to 8, wherein the test strip is a test strip for immunochromatography.

10. A container for housing a test strip and a desiccant agent, comprising:
a first room for housing the test strip; and
a second room for housing the desiccant agent and is communicated with the first room.

11. A manufacturing method for a test strip package comprising steps of:
providing a container assembly comprising a plurality of containers, wherein each container has a first room for housing a test strip and a second room for housing a desiccant agent;
putting the test strip in each of the first rooms of the container assembly and the desiccant agent in each of the second rooms of the container assembly; and
sealing the first and the second rooms of the container assembly so as to form a test strip package assembly.

12. The manufacturing method of Claim 11, further comprising
a step of separating a test strip package from the test strip package assembly.

13. The manufacturing method of Claim 11 or Claim 12, wherein
the container assembly is formed allowing separation of containers one by one.
